(19) Europäisches Patentamt
European Patent Office
Office européen des brevets

(11) Publication number: **0 235 259 B1**

(12) # EUROPEAN PATENT SPECIFICATION

(45) Date of publication of patent specification: **29.07.92**

(51) Int. Cl.5: **A61L 15/16**

(21) Application number: **86905556.6**

(22) Date of filing: **28.08.86**

(86) International application number:
**PCT/US86/01739**

(87) International publication number:
**WO 87/01291 (12.03.87 87/06)**

(54) **NOVEL TRANSDERMAL ANTI-ANGINAL PHARMACEUTICAL DOSAGE UNIT AND PROCESS FOR ITS ADMINISTRATION.**

(30) Priority: **30.08.85 US 770968**

(43) Date of publication of application:
**09.09.87 Bulletin 87/37**

(45) Publication of the grant of the patent:
**29.07.92 Bulletin 92/31**

(84) Designated Contracting States:
**AT BE CH DE FR GB IT LI LU NL SE**

(56) References cited:
**EP-A- 0 127 468**
**EP-A- 0 196 769**
**US-A- 3 742 951**
**US-A- 3 996 934**
**US-A- 4 291 015**
**US-A- 4 336 243**
**US-A- 4 405 616**
**US-A- 4 421 737**
**US-A- 4 485 087**
**US-A- 4 486 193**
**US-A- 4 505 891**
**US-A- 4 533 540**
**US-A- 4 542 013**
**US-A- 4 555 398**
**US-A- 4 562 075**
**US-A- 4 568 343**
**US-A- 4 573 996**
**US-A- 4 592 753**
**US-A- 4 608 249**

(73) Proprietor: **Rutgers, The State University**
**Old Oueens Bldg.**
**New Brunswick New Jersey 08903(US)**

(72) Inventor: **CHIEN, Yie, W.**
**5 West Lake Court**
**North Brunswick, NJ 08902(US)**
Inventor: **LEE, Chia-Shun**
**17 Forest Glen**
**Highland Park, NJ 08904(US)**

(74) Representative: **Calamita, Roberto et al**
**Frank B. Dehn & Co., Imperial House, 15-19 Kingsway**
**London WC2B 6UZ(GB)**

**EP 0 235 259 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a novel transdermal anti-anginal pharmaceutical absorption dosage unit comprising a backing layer; an intermediate adjoining layer of solid polymer matrix in which the anti-anginal pharmaceutical is dispersed; and a final biologically acceptable adhesive layer which is in communication with the solid polymer matrix layer and is adapted to adhere to the skin of a subject being administered an anti-anginal pharmaceutical, the adhesive layer having dispersed therein an effective amount of one or more skin permeation enhancing compounds for the nitroglycerin or other anti-anginal pharmaceutical dispersed in the polymer matrix layer.

It has previously been found that nitroglycerin and other anti-anginal pharmaceuticals are absorbed to a degree through the skin. This is referred to as transdermal absorption. Anti-anginal pharmaceuticals as used herein mean the nitrate and nitrite esters of mono- and poly-hydric alcohols which are pharmaceutically acceptable, have anti-anginal effectiveness, and are susceptible to transdermal absorption. One means of effecting transdermal absorption of an anti-anginal has been to distribute nitroglycerin within a polymeric disc or a container of a gel, which is brought into contact with an area of the skin of the subject to be treated with the nitroglycerin. Also, ointments or lotions containing nitroglycerin have been applied to an area of the skin of the subject to be treated. Problems encountered in such treatment include inadequate control over the rate and duration of transdermal absorption or the rate can be too slow in the case of certain dosage forms, especially from nitroglycerin-containing discs or nitroglycerin-containing gel container dosage units or pads. Nitroglycerin has been administered using tablet formulations by which the nitroglycerin is absorbed through the sublingual mucosa. Such sublingual mucosa absorption is rapid and effective to treat acute angina attacks, but does not provide sustained, constant blood levels of nitroglycerin together with long-term absorption. It has been found that the transdermal absorption rates of certain pharmaceuticals can be increased by use of absorption promoting compounds (also referred to as skin permeation enhancers) with the pharmaceutical to be absorbed when compounding in the polymeric disc or the pharmaceutical-containing gel.

It is desired to improve the dosage unit forms or devices by which anti-anginal pharmaceuticals are transdermally absorbed, especially in view of the importance of their administration by this means. Desired transdermal absorption would provide an avoidance of the large, unwanted destruction of anti-anginal pharmaceuticals by metabolism in the gastro-intestinal tract and "first-pass" hepatic elimination, which is, for example, usually about 90 percent or more of orally administered nitroglycerin. The transdermal absorption minimizes inter-and intra-patient variations regarding incompatibilities and metabolisms. By transdermal absorption, it is deemed possible to provide more constant concentration of the anti-anginal pharmaceuticals in the body and to realize a greater pharmaceutical efficiency. It is also possible, by proper transdermal absorption, to reduce the frequency of effective dosing. Transdermal administration provides most of the advantages of intravenous and sublingual dosing without most of the disadvantages of such dosing.

It is desired that improved transdermal absorption dosage unit forms and processes of transdermal administration be developed. A number of advantages would result.

This invention relates to an improved transdermal anti-anginal pharmaceutical containing dosage unit comprising:

a) a backing layer which is substantially impervious to the pharmaceutical to be delivered transdermally;

b) a polymeric matrix disc layer which is adhered to said backing layer and which has microdispersed therein an amount of an anti-anginally effective pharmaceutical selected from pharmaceutically acceptable nitrate and nitrite esters of mono- and poly-hydric alcohols and combinations thereof but not including nitroglycerin, which will provide a dosage amount of said pharmaceutical; and

c) an adhesive layer which is adhered to said polymer matrix disc layer, and which has distributed therein an effective amount of one or more skin permeation enhancers which provide substantial skin absorption enhancement for said pharmaceutical;

said dosage unit having an enhancing factor of at least 1.2.

The backing layer is made from materials that are substantially impermeable with regard to the anti-anginal pharmaceutical of the transdermal dosage unit. It can be made of polymers such as polyethylene, polypropylene, polyvinylchloride, polyesters such as poly(ethylene phthalate), and laminates of polymer films with metallic foils such as aluminum foil.

The polymer matrix disc layer is fabricated from biologically acceptable polymers. The polymer matrix disc layer which has the anti-anginal pharmaceutical distributed therein can suitably be made of a medical-grade silicone polymer such as a polydimethylsiloxane polymer, or another silicone polymer containing methylvinyl siloxane groups. The anti-anginal pharmaceutical is suitably dispersed in the silicone polymer,

to which mixture a curing agent is suitably added. The polymer-anti-anginal pharmaceutical mixture is then formed into a layer of an appropriate thickness and is cured. The matrix layer is adhered to the backing layer, which can be done directly. Other suitable polymers can be used in the formation of the polymer matrix disc layer which are elastomers or thermoplastics. Care must be taken that the polymer selected is compatible with the anti-anginal pharmaceutical, permits its release for transdermal absorption and is free or sufficiently free from any biologically unacceptable components.

Finally, the adhesive layer is applied to the polymer matrix disc layer. The skin permeation enhancer compound is mixed thoroughly with the adhesive polymer which is suitable for adhesion to the skin locus to which the transdermal matrix dosage unit will be applied. The adhesive polymer-skin permeation enhancer layer can be applied to the polymer matrix disc layer by coating or by solvent casting. Alternatively, part of the adhesive skin permeation enhancer layer can be applied to the inner surface of a release liner and the remainder can be applied to the matrix layer surface; the two adhesive-skin permeation enhancer surfaces then are pressed together to form a single layer. The adhesive polymer-skin permeation enhancer layer is desirably thin in the micron-range thickness, suitably 10-300 microns in thickness, desirably 20 to 250 microns, and preferably 50 to 200 microns in thickness.

The transdermal anti-anginal pharmaceutical absorption dosage units of this invention have an Enhancing Factor of at least 1.2, preferably at least 1.3, and more preferably at least about 2.0. Enhancing Factor is defined as the ratio of normalized permeation rate [in mcg/cm$^2$/hr] of a dosage unit of this invention with skin permeation enhancer/the normalized permeation rate of a corresponding dosage unit without enhancer in the adhesive layer.

The invention also provides a process for administering an anti-anginal pharmaceutical transdermally by forming the anti-anginal pharmaceutical-containing polymer matrix disc dosage unit having a polymer matrix disc layer which has the pharmaceutical dosage dispersed therein, to which matrix disc is adhered a skin permeation enhancer-containing adhesive layer and, by applying said dosage unit by way of said adhesive layer to the skin of the subject to be treated, whereby said pharmaceutical is transdermally administered to said subject to achieve desired systemic effects.

The backing layer can be made of any suitable material which is impermeable to the active substance of the polymer matrix layer. The backing layer serves as a protective cover for the matrix layer and provides also a support function. Examples of materials that are suitable are films of high and low density polyethylene, polypropylene, polyvinylchloride, polyesters such as poly(ethylene phthalate) and the like. Preferably, the materials for the backing layer are laminates of such polymer films with a metal foil such as aluminum foil. In such laminates, a polymer film of the laminate will usually be in contact with the polymer matrix layer. The backing layer can overlay the matrix and adhesive layer as desired for protection and to provide the desired pharmaceutical elegance of the final matrix dosage unit form. The protective layer can be any appropriate thickness which will provide the desired protective and support functions. A suitable thickness will be from 10 to 200 microns. Desirably, the thickness will be from 20 to 150 microns, and preferably be from 30 to 100 microns. The polymer matrix layer can be made from silicone elastomers of the general polydimethylsiloxane structure, such as silicone polymers of the following formula:

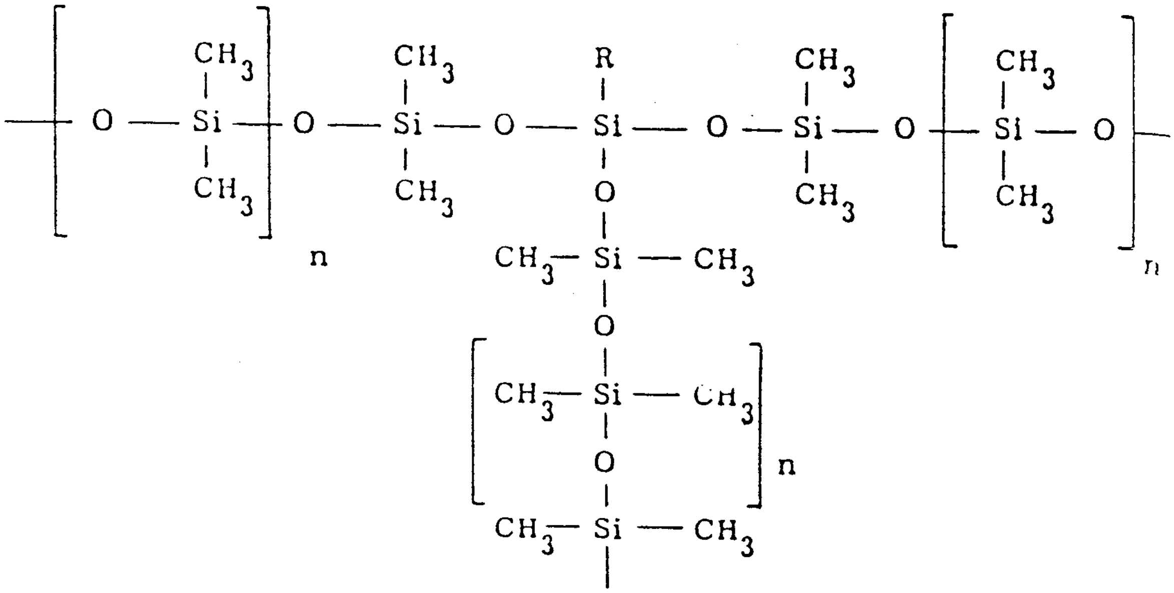

wherein R is alkyl or alkoxy containing 1-7 carbon atoms, vinyl or phenyl and wherein n is 100-5000.

The silicone polymers selected preferably are cross-linkable at moderate temperatures such as at room temperature, using cross-linking catalysts which are biologically acceptable in the final polymer matrix and which are compatible with the anti-anginal pharmaceutical to be used in making the polymer matrix dosage forms. Some suitable siloxane polymers are crosslinkable copolymers having dimethyl and methylvinyl siloxane which can be cross-linked as by using a suitable peroxide catalyst or a suitable tin or platinum catalyst system. Other cross-linking sites can be present in the polysiloxane elastomers used. Suitable siloxane medical-grade polymers are sold under the designations Silastic 382, Q7-4650, Q7-4665, Q7-4735, Q7-4750, Q7-4765 and MDX-4-4210. "Silastic" is a Registered trade mark.

Generally, polymers used to form the biologically acceptable polymer matrix are those capable of forming a thin layer of coatings or a disc of drug-dispersing matrix through which the anti-anginal pharmaceutical can pass at a controlled rate. Suitable polymers are biologically-acceptable and compatible with the pharmaceutical, non-allergenic and insoluble in and non-irritating to body fluids or tissues with which the device is contacted. The use of soluble polymers is to be avoided since dissolution or erosion of the matrix would affect the release rate of the anti-anginal pharmaceutical as well as the capability of the dosage unit to remain in place for convenience of removal.

Exemplary materials for fabricating the biologically acceptable polymer matrix include polyethylene, polypropylene, ethylene/propylene copolymers, ethylene/ethylacrylate copolymers, ethylene/vinyl acetate copolymers, silicone elastomers, especially the medical-grade poly-dimethylsiloxanes, neoprene rubber, chlorinated polyethylene, polyvinyl chloride; vinyl chloride-vinyl acetate copolymer, polymethacrylate polymer (hydrogel), polyvinylidene chloride, poly(ethylene terephthalate), butyl rubber, epichlorohydrin rubbers, ethylene-vinyl alcohol copolymer, ethylenevinyloxyethanol copolymer; silicone copolymers, for example, silicone-polycarbonate copolymers; cellulose polymers, for example methyl or ethyl cellulose hydroxypropyl methyl cellulose, and cellulose esters; polycarbonates; polytetrafluoroethylene; and the like. For best results, the biologically acceptable polymer matrix should be selected from polymers with glass transition temperatures below room temperature. The polymer may, but need not necessarily, have a degree of crystallinity at room temperature. Cross-linkable monomeric units or sites can be incorporated into such polymers. For example, cross-linking monomers can be incorporated into polyacrylate polymers, which provide sites for cross-linking the matrix after microdispersing the anti-anginal pharmaceuticals into the polymer. Known cross-linkable monomers for polyacrylate polymers include polymethacrylic esters of polyols such as butylene diacrylate and dimethacrylate, trimethylol propane trimethacrylate and the like other monomers which provide such sites include allyl acrylate, allyl methacrylate, diallyl maleate and the like.

The adhesive layer is suitably made using a silicone adhesive, such as a polydimethylsiloxane adhesive of the following formula:

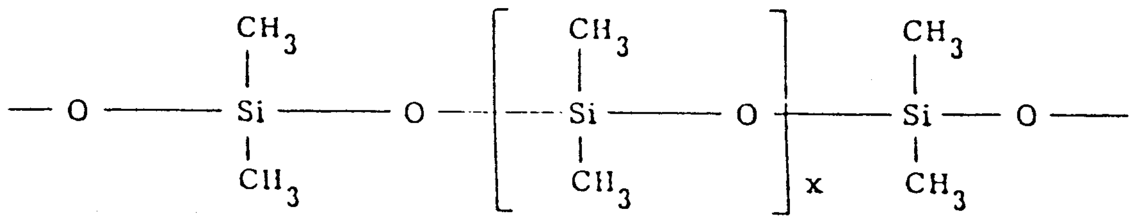

wherein x shows the unit is repeated to the extent to provide desired properties.

For example, adhesive products sold by Dow Corning under the designation DC-355 are suitable in making the adhesive layer. The adhesive polymer must be biologically acceptable and compatible with the active substance and skin permeation enhancer used. Certain poly-acrylate adhesive polymers (in the form of an alkyl ester, amide, free acid, or the like) can also be used with anti-anginal pharmaceuticals. Other suitable hypoallergenic pressure-sensitive contact adhesive compositions are also known. A preferred adhesive layer is pressure-sensitive.

The adhesive layer then is finally covered with a releaseable protective layer liner which is made from materials which are substantially impermeable to the anti-anginal pharmaceuticals used, the skin permeation enhancer used and any other components of the polymer matrix dosage unit. The polymer materials and metal foil laminates used for the backing layer can be used to make the protective layer, provided the layer is made strippable or releasable such as by applying conventional siliconizing.

In making the anti-anginal pharmaceutical containing polymer matrix disc layer, silicone elastomers such as polydimethylsiloxane of the formula described above can be suitably used. In making such polymer matrix disc dosage units, it has been found suitable to use lactose as a dispersing agent to stabilize the nitroglycerin. Other suitable dispersing agents can also be used to replace lactose as long as they can produce a stable dispersion. A dispersing agent might be unnecessary if the anti-anginal pharmaceutical is a solid. Depending upon the drug loading desired, a suitable amount of a dispersing agent has been found to be 1-9 equivalents (by weight) based on the weight of active substance. The blend of active substance with dispersing agent then is added to the polymer used to make the matrix disc layer. The amount of active substance added depends upon the amount of active substance dosage desired in each dosage unit and the amount which can be incorporated into the polymer matrix disc to retain suitable structural, diffusion and other properties in the final matrix disc. It has been found, for example, that up to 30 parts of nitroglycerin dispersion can be satisfactorily added to 70 parts of the polymer used in making the matrix disc, such as silicone elastomers. The mixture of the polymer and nitroglycerin dispersing agent is then thoroughly mixed using a high-torque mixer to form a homogeneous microdispersion of nitroglycerin in the polymer. With continued agitation, an amount of cross-linking catalyst is desirably added together with relatively low molecular weight polymer having a compatible chemical structure. For example, when polydimethylsiloxane is used as the polymer, a relatively low molecular weight polydimethylsiloxane and a cross-linking catalyst is added (such as 10 parts by weight of the low molecular weight polydimethylsiloxane and 30 drops of stannous octanoate per 100g. amount of the final polydimethylsiloxane-nitroglycerin mixture) to the above illustrative composition of 20 parts of nitroglycerin dispersion and 70 parts of polydimethylsiloxane polymer. Again, the mixture is agitated with a high-torgue mixer to form a uniform admixture. After each mixing step, the composition is subjected to vacuum to remove any entrapped air.

It is also desirable to add an amount of dextran to the polymeric mixture used in making the matrix, if it is compatible with the polymer used. It has been found useful to incorporate about one to about ten parts by weight based on the polymer of dextran, preferably about three to about eight parts by weight, and usually about six parts by weight being a preferable amount depending upon the polymer and anti-anginal pharmaceutical used.

The deaerated mixture is then placed in a device maker and heated to suitable elevated temperature to promote cross-linking. A suitable temperature for cross-linking when the polymer used is polydimethylsiloxane of the above formula and the cross-linking catalyst is stannous octanoate, is from 10°C to 200°C, desirably 20°C to 100°C. The temperature used should not cause significant degradation of the active substance. The polymer matrix sheet desirably is 0.05 to 5 mm, preferably 0.1 to 3 mm in thickness. The resulting cross-linked polymer matrix sheet is removed from the device maker and can be cut to form discs with desired shapes and sizes. The discs are then attached to a backing sheet, as described above, using an area, suitably 5 to 100 sq. cm., preferably, 8 to 80 sq. cm., generally 10 to 60 sq. cm. being more preferable. The shape of the discs can vary; they can be circular, square, rectangular, or other desired shapes.

The polymer matrix disc layer, generally speaking, should contain some excess amount of the dispersed active substance over the dosage amount desired to be transdermally absorbed by the subject to be treated. Ordinarily, this excess is small, such as less than 2-fold excess. Generally speaking, an amount of the active substance used, which is sufficient, is less than 2 to 10 times the desired dosage to less than 2 to 5 times, the desired dosage to be transdermally absorbed being adequate, depending upon the physiochemical properties of nitroglycerin, as well as the nature of the polymer of the matrix disc layer and other factors. The amount of active substance loading in the matrix can be varied, depending upon the polymer used in making the matrix layers, the dosage desired, the skin permeation enhancer system or systems used and the like. Ordinarily, however, in using silicone polymers for making the matrix layer, use of up to about 10 percent loading of active substances is adequate. It has been observed that a greater loading does not assure a greater transdermal absorption of nitroglycerin and at least at times results in no significant increase.

The adhesive polymer layer containing the skin permeation enhancer is made as by dissolving the enhancer compound in a solvent for the enhancer which is compatible with the adhesive polymer solution used to make the adhesive layer containing the skin permeation enhancer. Any suitable amount of solvent can be used as necessary to dissolve the quantity of enhancer to be admixed with the adhesive polymer solution used. For example, 3 to 10 parts of solvent can be used to dissolve one part of skin permeation enhancer, depending upon the solubility of the enhancer. When using polydimethylsiloxane adhesive solution, it has been found suitable to use 2 to 30 parts of skin permeation enhancer in 20 to 50 parts of solvent (such as acetone, methyl ethyl ketone, ethyl acetate or other suitable solvent) and add the solution to 100 parts of the adhesive solution. The enhancer-adhesive combination is thoroughly mixed and a coating thereof is applied using a film coating machine to the matrix disk layer or to a strippable release liner, as described above. Preferably, in order to assure adequate adhesion of the adhesive polymer layer to the skin of the subject treated, an enhancer-adhesive polymer solution having a relatively low concentration of enhancer, e.g., 1-2 percent based on the weight of the adhesive polymer is used to apply a coating to the release liner. The thickness of this coating ordinarily is a minor percentage of the thickness of the final adhesive layer, such as 20-40 percent of the total adhesive polymer layer. The remainder of the adhesive polymer layer having a suitable higher concentration of the enhancer is used to coat the matrix disc layer. Suitable higher concentrations of enhancer are usually 10 to 30 percent based on the adhesive polymer weight, depending on solubility, desired final amount of skin enhancer agent and other factors. The solvent of the respective coatings is removed by evaporation. The respective coatings are combined to make the final adhesive polymer-enhancer agent layer by application of constant pressure.

A suitable release liner being a poly(ethylene phthalate) laminated with aluminum foil. The poly(ethylene phthalate) side to which the adhesive-enhancer coating is applied, is made strippable to conventional siliconizing or by other suitable means, such as fluorocarbon-coating. The thickness of the adhesive-enhancer layer normally applied is 10 to 200 microns, preferably 30 to 150 microns. The amount of enhancer in the adhesive layer depends in part on the rapidity at which it is desired that the active substance be absorbed. Generally speaking, 1 to 30 percent of skin permeation enhancer based on the weight of the adhesive is suitable depending upon the enhancer, matrix polymer, adhesive and other factors. Desirably, 2 to 20 percent of skin permeation enhancers are used depending upon the above recited factors. The adhesive layer containing the skin permeation enhancer is applied to the polymer matrix disc surfaces by application of a constant pressure.

The four-layer transdermal polymer matrix dosage units are excised. The backing layer as desired can be shaped around the sides of the dosage unit including the polymer matrix layer if such protection is desired. The resulting polymer matrix dosage unit forms are then placed in appropriate storage until they are to be applied in transdermal treatment.

The pharmaceutical active substance is dispersed in the polymer matrix disc layer. Another type of pharmaceutical may also be dispersed in the polymer matrix disc layer, which includes any pharmaceutical which is capable of being transdermally or topically administered to a subject to be treated and which does not materially interfere with the desired active substance absorption and treatment. Such additional pharmaceutical used should have a daily effective dose of less than about 100 mg. With the controlled release of active substance and any additional pharmaceutical at a relatively steady-state rate over a prolonged period, typically 24 hours or longer, the patient is provided with the benefit of a steady infusion of the pharmaceutical component over a prolonged period.

It will be appreciated that the active substance may be added to the above mixture not only in the form of the pure chemical compound, but also in admixture with other pharmaceuticals which may be transdermally applied or with other ingredients which are not incompatible with the desired objective of transdermally administering the active substance to a patient. Thus, it will be suggested to those in the art

of angina treatment to consider substitution in part or in total other anti-anginal nitrate or nitrite compounds or other pharmaceuticals which have anti-anginal treatment properties. Anti-anginal pharmaceuticals used in this invention are nitrate or nitrite esters of mono-or polyhydric alcohols which are pharmaceutically acceptable, which have anti-anginal effectiveness and which are susceptible to transdermal absorption and can be selected from but are not limited to isosorbide dinitrate, pentaerythrityl tetranitrate, erythrityl tetranitrate, amyl nitrite and the like.

The skin permeation enhancers which can be used in carrying out this invention can vary. Ones that give preferred results with the polymer matrix dosage unit form can vary. In some instances, the use of permeation enhancer in making a polymer matrix dosage form will result in good or even excellent absorption of active susbtance, might result in relatively low enhancement when another dosage unit form of the invention is used. Use of combinations of two or more of the skin permeation enhancer compounds frequently result in superior results, such as greater transdermal absorption. Some amount of skin permeation enhancer can also be incorporated into the polymer matrix layer, if desired, the amount used in the matrix layer can be varied so long as it is effective, such as an amount equivalent to the amount used in making the adhesive layer or an amount which is 20-80% of the concentration used in the adhesive layer. The skin permeation enhancers can be varied if desired in the respective layers.

Specific skin permeation enhancers which can be used in making the polymer matrix dosage forms of this invention include saturated and unsaturated fatty acids and their esters, alcohols, monoglycerides, acetates, diethanolamides and N,N-dimethylamides, such as oleic acid, capric or decanoic acid, propyl decanoate, propyl or isopropyl oleate, oleyl acetate, propyl or isopropyl myristate, myristyl alcohol, myristyl N,N-dimethyl amide, stearic acid and stearyl alcohol, propyl stearate monostearin, and combinations of them with, for example, 1-dodecylazacycloheptan-2-one sold under the trademark Azone by Nelson Research and Development; decyl methyl sulfoxide, dimethyl sulfoxide, salicylic acid and derivatives, N,N-diethylm-toluamide, crotamiton, 1-substituted azacycloalkan-2-ones such as disclosed in U. S. Patent 4,316,893 (the 1-substituent having 0-17 carbon atoms, preferably, 1-11 carbon atoms), squalane and various other compounds which are biologically compatible and have transdermal permeation activity. It has been found useful to incorporate menthol with one or more skin permeation enhancers, even though it is not currently certain whether menthol alone has skin permeation enhancer activity. Ethyl alcohol and other short chain alkanols (with 1-4 carbon atoms) which have substantially the same properties and activity as ethyl alcohol do not come within the definition of skin permeation enhancer as used herein.

Of the following Examples, Examples 1-3 relate to nitroglycerin as active substance which does not fall within the invention as claimed, and Example 14 relates to active substances falling within the invention as claimed.

EXAMPLE 1

A dispersion of 10 parts by weight each of pure nitroglycerin oil and lactose is made by using a high-torque mixer (sold by Cole-Parmer Company). The nitroglycerinlactose dispersion is homogeneously dispersed in 70 parts of silicone elastomer using the high-torque mixer and at about 1,000 rpm. The silicone elastomer is a polydmethylsiloxane polymer sold by Dow Corning Company under the designation Silastic Medical Grade 382 Elastomer ("Silastic" is a regd. Trade Mark). The elastomer is believed to have the following structural formula:

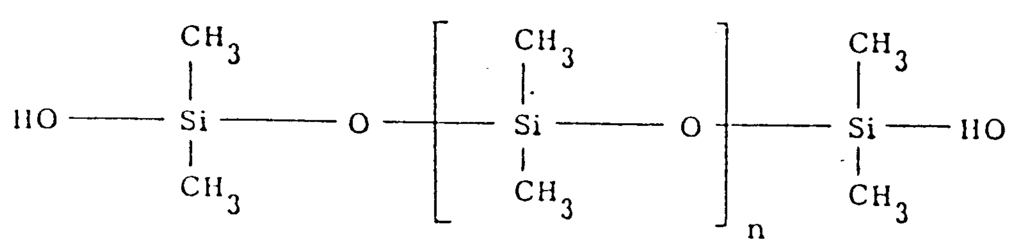

wherein n indicates the number of repeating units.

With continued agitation, 10 parts of DC-360 (silicone medical fluid) and 30 drops (for every 100 g of the mixture) of a cross-linking agent designated as catalyst M is added, which is stannous octanoate. After each addition of the material, the mixture is thoroughly mixed and is placed under vacuum to remove entrapped air.

The nitroglycerin-polydimethylsiloxane dispersion is placed into a device maker and is cross-linked at room temperature or at an elevated temperature (60° - 100°C) to form a cross linked, medicated polymer sheet, which has a thickness of 0.2-2 mm.

The medicated polymer sheet is removed from the device maker and is cut into circular discs of about 3-20 sq. cm. The discs are attached to a backing layer of heat sealable polyester film which is laminated to aluminum foil. This laminate is sold by 3M Company as Scotchpak 1006.("Scotchpak" is a reg.d Trade Mark). The medicated discs are attached to the backing layer using an adhesive polymer solution, a silicone adhesive polymer sold by Dow Corning as DC-355 or medical-grade adhesive A being suitable. Alternately, the discs can be formed directly on the backing layer and in practice are.

The skin permeation enhancer-adhesive layer is made by dissolving the necessary weight of a skin permeation enhancer in 30 parts of acetone. The acetone solution then is added to 100 parts of a silicone adhesive solution sold by Dow-Corning under the designation DC-355. The mixture is thoroughly mixed to form a homogeneous mixture of skin permeation enhancer and adhesive polymer.

The adhesive polymer layer is formed by making multiple coatings. Desirably, a coating of adhesive polymer solution containing 1-2 percent enhancing agent is applied to the release liner (a fluorocarbon-coated polyester film). This lower concentration of enhancing agent aids assurance that the surface of the final adhesive layer when applied to skin of the subject treated will satisfactorily adhere. The remaining portion of the adhesive coating necessary to make up the final adhesive layer thickness has a higher enhancing agent content (10-30 percent) and is applied to the matrix layer. The solvent of the respective coatings is removed by evaporation. The release liner so-coated is then applied to the adhesive coated matrix layer under a constant pressure to provide a firmly adhered strip of a four-layered structure as follows:

1. Backing layer
2. Nitroglycerin-containing polymer matrix layer
3. Skin permeation enhancer-adhesive layer (50-200 micron thickness)
4. Release film layer which can be readily removed to permit application to the skin of the subject to receive transdermally the nitroglycerin.

By use of an appropriate cutter, the strip is cut into a suitable shape such as circular to provide the transdermal nitroglycerin polymer matrix dosage units which have an area of about 20 sq. cm.

The above polymer matrix disc dosage units are made using the following skin permeation enhancers as shown in the TABLES.

Use of tert-butyl alcohol did not function as a skin permeation enhancer but rather the dosage units using tert-butyl alcohol resulted in less nitroglycerin absorption than the absorption from a controlled unit in which no enhancer compound was added to the adhesive layer.

The transdermal absorption of nitroglycerin from the nitroglycerin polymer matrix dosage units of this invention is evaluated by use of skin from a "hairless" mouse or human cadaver by following the procedure described by P. R. Keshary and Y. W. Chien, in Drug Develop. & Ind. Pharm., 10 (6) 883-913(1984).

The following TABLES show the transdermal absorption of nitroglycerin from the nitroglycerin-containing polymer matrix disc dosage units made by the above procedure:

TABLE I

SKIN PERMEATION ENHANCEMENT OF

NITROGLYCERIN BY VARIOUS ENHANCERS

| Dosage Unit | Agents[1] (1.6 mg/cm²) | Permeation Rate (mcg/cm²/hr± S.D.) | Enhancing[2] Factors |
|---|---|---|---|
| 1. | None | 25.72 ± 5.41 | 1.00 |
| 2. | Stearic Acid | 27.26 ± 2.53 | 1.06 |
| 3. | Stearyl Alcohol | 25.38 ± 0.05 | 0.99 |
| 4. | Stearyl Propyl Ester | 42.91 ± 4.81 | 1.67 |
| 5. | Mono-Stearin | 36.82 ± 0.01 | 1.43 |
| 6. | Oleic Acid | 70.55 ± 11.06 | 2.74 |
| 7. | Oleyl Alcohol | 61.74 ± 5.83 | 2.40 |
| 8. | Oley Propyl Ester | 62.40 ± 11.12 | 2.43 |
| 9. | Mono-Olein | 51.14 ± 10.52 | 1.99 |
| 10. | Myristic Acid | 28.04 ± 1.33 | 1.09 |
| 11. | Myristyl Alcohol | 45.72 ± 2.33 | 1.78 |
| 12. | Myristyl Propyl Ester | 49.46 ± 5.62 | 1.92 |
| 13. | Mono-Myristein | 22.54 ± 2.90 | 0.88 |
| 14. | *n*-Decyl Alcohol | 65.59 ± 11.39 | 2.55 |
| 15. | Decyl Acetate | 40.01 ± 5.28 | 1.56 |
| 16. | 1-Dodecylazacyclo-heptan-2-One | 35.83 ± 3.90 | 1.39 |
| 17. | Decyl Methyl Sulfoxide | 56.68 ± 1.82 | 2.20 |
| 18. | T-Butanol | 22.73 ± 2.02 | 0.88 |
| 19. | 1-Propanol | 27.56 ± 3.41 | 1.07 |
| 20. | 2-Propanol | 27.06 ± 4.69 | 1.05 |
| 21. | Triethanolamine | 28.62 ± 4.79 | 1.11 |
| 22. | Malonic Acid Diethyl Ester | 40.12 ± 2.20 | 1.56 |
| 23. | Maleic Acid Diethyl Ester | 30.01 ± 3.83 | 1.17 |
| 24. | Mandelic Acid Ethyl Ester | 27.09 ± 0.20 | 1.05 |
| 25. | Glycylglycine | 36.94 ± 4.36 | 1.44 |

1) Contained in the adhesive layer at a surface concentration of 1.6 mg/cm².

2) Enhancing Factor = $\frac{\text{(Normalized Permeation Rate) Enhancer}}{25.72}$

TABLE II

EFFECT OF LOCATION AND CONCENTRATION OF PROPYL OLEATE, AS SKIN PERMEATION ENHANCER, ON TRANSDERMAL ABSORPTION OF NITROGLYCERIN

| Concentration of Enhancer in | | Normalized Permeation Rate | Enhancing |
|---|---|---|---|
| Polymer Matrix | Adhesive Layer | (mcg/cm²/hr ± S.D.) | Factor* |
| 0.0% | 0.0% | 40.33 ± 6.04 | 1.00 |
| 2.5% | - | 46.09 ± 5.07 | 1.14 |
| - | 2.5% | 59.68 ± 17.34 | 1.48 |
| 2.5% | 2.5% | 71.00 ± 14.48 | 1.76 |
| 5.0% | - | 65.86 ± 22.61 | 1.63 |
| - | 5.0% | 87.72 ± 16.31 | 2.18 |
| 5.0% | 5.0% | 109.58 ± 10.03 | 2.72 |
| - | 10.0% | 125.86 ± 12.21 | 3.12 |

* Enhancement Factor = $\frac{\text{(Normalized Permeation Rate) Enhancer}}{40.33}$

TABLE III

| EFFECT OF SKIN PERMEATION ENHANCER CONCENTRATION IN ADHESIVE LAYER ON THE ENHANCEMENT OF TRANSDERMAL ABSORPTION OF NITROGLYCERIN | | | | | | | | |
|---|---|---|---|---|---|---|---|---|
| Enhancing Conc. ($mg/cm^2$) | Enhancing Factor | | | | | | | |
| | A* | B | C | D | E | F | G | H |
| 0 | 1.00 | 1.00 | 1.00 | 1.00 | 1.00 | 1.00 | 1.00 | 1.0 |
| 0.08 | 1.16 | -- | -- | -- | -- | -- | -- | - |
| 0.20 | 1.41 | -- | -- | -- | -- | -- | -- | - |
| 0.37 | 1.68 | -- | -- | -- | -- | -- | -- | - |
| 0.66 | 2.41 | -- | -- | -- | -- | -- | -- | - |
| 0.80 | -- | 2.59 | 1.67 | 1.22 | 1.69 | 1.56 | 1.61 | 1.3 |
| 0.96 | 1.99 | -- | -- | -- | -- | -- | -- | - |
| 1.27 | 1.41 | -- | -- | -- | -- | -- | -- | - |
| 1.59 | 1.44 | 4.11 | 1.92 | 2.43 | 2.82 | 2.58 | 1.87 | 2.0 |
| 2.40 | -- | -- | -- | -- | -- | -- | 2.02 | - |
| 3.18 | 1.51 | 4.41 | 3.07 | 3.08 | 2.65 | 1.50 | -- | 1.6 |
| 4.78 | -- | -- | 3.41 | 3.78 | -- | 1.74 | -- | 1.5 |
| 5.00 | -- | -- | -- | -- | -- | -- | 1.36 | - |
| 10.00 | -- | -- | -- | -- | -- | -- | 1.22 | - |

*Enhancer
A = 1-Dodecylazacycloheptan-2-one
B = Decanoic acid
C = Propyl myristate
D = Propyl oleate
E = Oleyl alcohol
F = 1-monolauroyl-rac-glycerol
G = Decylmethyl sulfoxide
H = Myristic acid, N,N-dimethylamide

TABLE IV

| EFFECT OF ENHANCER CONCENTRATION ON SKIN PERMEATION ENHANCEMENT OF NITROGLYCERIN BY COMBINATION OF ENHANCERS | | | | |
|---|---|---|---|---|
| Concentration of Enhancers in Adhesive Layer* (mg/cm$^2$) | | | | Enhancing Factor |
| A | B | C | D | |
| 1.27 | -- | -- | -- | 1.41 |
| 1.27 | 1.27 | -- | -- | 2.15 |
| 1.27 | -- | 1.27 | -- | 1.63 |
| -- | 1.27 | 1.27 | -- | 1.28 |
| 1.27 | 1.27 | 1.27 | -- | 2.37 |
| 0.34 | 3.2 | -- | -- | 2.34 |
| 0.34 | -- | 3.2 | -- | 2.81 |
| 0.34 | -- | -- | 2.3 | 1.87 |
| 0.17 | 1.6 | -- | -- | 1.84 |
| 0.17 | -- | 1.6 | -- | 1.85 |
| 0.17 | -- | -- | 1.09 | 1.35 |
| 0.17 | 0.83 | 0.83 | -- | 1.87 |
| 0.17 | 0.83 | -- | 0.79 | 2.83 |
| 0.17 | -- | 0.83 | 0.79 | 1.78 |
| 0.085 | 0.83 | 0.83 | 0.55 | 2.34 |

*Enhancer
A = 1-Dodecylazacyloheptan-2-one
B = Propyl oleate
C = Propyl myristate
D = Decylmethyl sulfoxide

TABLE V

| SKIN PERMEATION ENHANCEMENT OF NITROGLYCERIN BY COMBINATION OF ENHANCERS | | | | |
|---|---|---|---|---|
| Enhancers | Conc. ($mg/3.14cm^2$) | Permeation Rate ($mcg/cm^2/hr$ + S.D.) | Enhancing Factor | |
| | | | A[1] | B[2] |
| None | | 30.84 ± 1.32 | 1.00 | 1.00 |
| 1-Dodecylazacycloheptan-2-one | 1.0 | 42.68 ± 3.07 | 1.38 | 1.00 |
| +Propyl Myristate | 5.0 | 63.83 ± 2.23 | 2.07 | 1.50 |
| | 10.0 | 88.54 ± 1.31 | 2.87 | 2.07 |
| +Propyl Oleate | 5.0 | 69.00 ± 2.76 | 2.24 | 1.62 |
| | 10.0 | 83.50 ± 3.87 | 2.71 | 1.96 |
| +Myristyl Acetate | 5.0 | 116.01 ± 25.71 | 3.76 | 2.72 |
| | 10.0 | 103.89 ± 18.41 | 3.37 | 2.43 |
| +Oleyl Acetate | 5.0 | 89.71 ± 0.64 | 2.91 | 2.10 |
| | 10.0 | 107.03 ± 2.14 | 3.47 | 2.51 |
| +Glycylglycine | 5.0 | 47.30 ± 0.90 | 1.53 | 1.11 |
| +Decylmethyl Sulfoxide | 5.0 | 81.17 ± 7.11 | 2.63 | 1.90 |
| 1-Dodecylazacycloheptan-2-one | 2.0 | 46.71 ± 3.39 | 1.51 | 1.00 |
| +Propyl Myristate | 5.0 | 71.58 ± 14.57 | 2.32 | 1.53 |
| | 10.0 | 82.15 ± 0.05 | 2.66 | 1.76 |
| +Propyl Oleate | 5.0 | 50.20 ± 2.62 | 1.63 | 1.07 |
| | 10.0 | 79.90 ± 18.95 | 2.59 | 1.71 |
| +Salicylic Acid | 5.0 | 52.11 ± 0.40 | 1.69 | 1.12 |
| | 10.0 | 41.15 ± 6.80 | 1.33 | 0.88 |

1-Enhancing Factor A = normalized permeation rate defined skin permeation enhancer content/normalized permeation rate of a corresponding dosage unit with no enhancer.
2-Enhancing Factor B = normalized permeation rate with defined combination of skin permeation enhancer content/normalized permeation rate of a corresponding dosage unit with only the defined amount of the enhancer of the combination, 1-dodecylazacycloheptan-2-one.

TABLE VI

| SKIN PERMEATION ENHANCEMENT OF NITROGLYCERIN BY COMBINATION OF 1-DODECYLAZACYCLOHEPTAN-2-ONE WITH OTHER SKIN PERMEATION ENHANCERS | | | | | | | |
|---|---|---|---|---|---|---|---|
| Concentration of Enhancers in Adhesive Layer* | | | | | | | Enhancing (mg/cm$^2$) Factor |
| A | B | C | D | E | F | G | |
| 0.0 | 0.0 | 0.0 | 0.0 | 0.0 | 0.0 | 0.0 | 1.00 |
| 0.34 | -- | -- | -- | -- | -- | -- | 1.18 |
| 0.34 | 0.8 | -- | -- | -- | -- | -- | 2.61 |
| 0.34 | 1.6 | -- | -- | -- | -- | -- | 4.43 |
| 0.34 | -- | 1.6 | -- | -- | -- | -- | 3.82 |
| 0.34 | 0.8 | 0.8 | -- | -- | -- | -- | 4.38 |
| 0.34 | 0.8 | 1.6 | -- | -- | -- | -- | 3.61 |
| 0.34 | -- | 0.8 | 0.8 | -- | -- | -- | 3.05 |
| 0.34 | -- | -- | -- | 1.6 | -- | -- | 2.47 |
| 0.34 | 0.8 | -- | -- | 0.8 | -- | -- | 4.33 |
| 0.34 | 0.8 | -- | -- | 1.6 | -- | -- | 4.31 |
| 0.34 | -- | 0.8 | -- | -- | 0.8 | -- | 2.69 |
| 0.34 | 0.8 | -- | -- | -- | -- | 0.8 | 2.22 |

*Enhancer
A = 1-Dodecylazacycloheptan-2-one
B = Decanoic acid
C = Decanol
D = Oleic acid
E = Oleyl alcohol
F = Lauric acid
G = Decyl methyl sulfoxide

TABLE VII

| SKIN PERMEATION ENHANCEMENT BY VARIOUS GLYCERIDES | | | |
|---|---|---|---|
| Glycerides (1.6 mg/cm$^2$) | Skin Permeation Rate (mcg/cm$^2$/hr) | | Enhancing Factor |
| | With | Without | |
| 1-Monolauroyl-RAC-Glycerol | 88.02 ± 19.71 | 34.1 ± 1.62 | 2.58 |
| 1-Monomyristoly-RAC-Glycerol | 29.88 ± 3.84 | 34.1 ± 1.62 | 0.88 |
| 1-Monopalmitoyl-RAC-Glycerol | 29.39 ± 1.76 | 34.1 ± 1.62 | 0.86 |
| 1,2-Dimyristoyl-RAC-Glycerol | 40.64 ± 4.27 | 39.4 ± 4.32 | 1.03 |
| 1,3-Dimyristoyl-RAC-Glycerol | 32.13 ± 6.94 | 34.1 ± 1.62 | 0.94 |
| 1,2,3-Trimyristoyl-RAC-Glycerol | 32.36 ± 4.61 | 39.4 ± 4.32 | 0.82 |

TABLE VIII

SKIN PERMEATION ENHANCEMENT OF NITROGLYCERIN BY UNSATURATED FATTY ACID ESTERS

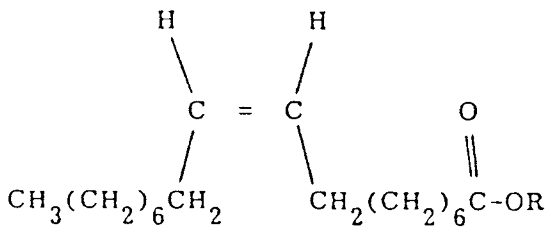

| Enhancers (1.6 mg/cm²) | R | Normalized Permeation Rate* (mcg/cm²/hr±S.D.) | Enhancing Factor |
|---|---|---|---|
| Methyl Oleate | $CH_3$ | 77.56 ± 3.54 | 2.62 |
| Ethyl Oleate | $CH_3CH_2$ | 68.48 ± 9.77 | 2.31 |
| Propyl Oleate | $CH_3CH_2CH_2$ | 71.96 ± 12.82 | 2.43 |
| Butyl Oleate | $CH_3CH_2CH_2CH_2$ | 61.00 ± 3.22 | 2.06 |
| Oleyl Acetate | $CH_3C$ | 69.05 ± 8.88 | 2.33 |

*Normalized Permeation Rate Without Enhancer = 29.66 ± 0.83 for Nitroglyceri

TABLE IX

SKIN PERMEATION ENHANCEMENT OF NITROGLYCERIN BY UNSATURATED FATTY ACID PROPYL ESTERS

| Enhancing Agents | Skin Permeation Rate ($mcg/cm^2/hr \pm$ S.D.) With | Skin Permeation Rate ($mcg/cm^2/hr \pm$ S.D.) Without | Enhancing Factor |
|---|---|---|---|
| Monoenoic Acid | | | |
| Oleic Acid Propyl Ester | 72.81 ± 12.97 | 30.01 ± 4.29 | 2.43 |
| Dienoic Acid | | | |
| Linoleic Acid Propyl Ester | 73.89 ± 16.03 | 30.01 ± 4.29 | 2.46 |
| Tetraenoic Acid | | | |
| Arachidonic Acid Prop.. Ester | 70.71 ± 31.76 | 30.01 ± 4.29 | 2.30 |

## TABLE X

### SKIN PERMEATION ENHANCEMENT OF NITROGLYCERIN BY SATURATED FATTY ACID AMIDE

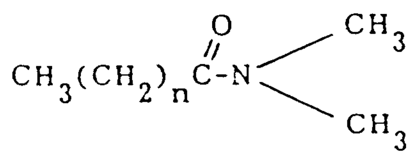

| Enhancers (1.6 mg/cm²) | n | Normalized Permeation Rate* (mcg/cm²/hr±S.D.) | Enhancing Factor |
|---|---|---|---|
| N-Caproic Acid N,N-Dimethylamide | 4 | 33.49 ± 4.12 | 1.21 |
| Lauric Acid N,N-Dimethylamide | 10 | 47.33 ± 1.76 | 1.71 |
| Myristic Acid N,N-Dimethylamide | 12 | 55.83 ± 7.39 | 2.02 |

*Normalized Permeation Rate Without Enhancer = 27.65 ± 2.47 for Nitroglycerin.

TABLE XI

SKIN PERMEATION ENHANCEMENT OF NITROGLYCERIN BY VARIOUS MYRISTYL DERIVATIVES

| Myristyl Derivative | Skin Permeation Rate ($mcg/cm^2/hr \pm$ S.D.) With | Without | Enhancing Factor |
|---|---|---|---|
| Myristic Acid | 31.24 ± 1.48 | 28.66 ± 2.72 | 1.09 |
| Myristate Sodium | 36.28 ± 4.06 | 39.38 ± 4.32 | 0.92 |
| Myristyl Esters: | | | |
| Methyl | 63.36 ± 8.66 | 32.52 ± 3.52 | 1.95 |
| Ethyl | 52.40 ± 1.47 | 28.66 ± 2.72 | 1.83 |
| Propyl | 67.77 ± 7.70 | 35.24 ± 1.75 | 1.92 |
| Isopropyl | 61.29 ± 4.49 | 35.24 ± 1.75 | 1.74 |
| Myristyl | 40.30 ± 0.03 | 28.86 ± 0.91 | 1.40 |
| Myristyl Alcohol | 51.30 ± 2.62 | 28.86 ± 0.91 | 1.78 |
| Myristyl Acetate | 58.54 ± 3.88 | 28.86 ± 0.91 | 2.03 |
| Myristyl N,N-Dimethylamide | 57.87 ± 7.66 | 28.86 ± 2.72 | 2.02 |

## TABLE XII

### ENHANCEMENT IN TRANSDERMAL ABSORPTION OF NITROGLYCERIN BY SATURATED FATTY ACIDS

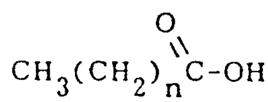

| Enhancers (1.6 mg/cm²) | n | Normalized Permeation Rate (mcg/cm²/hr±S.D.) | Enhancing Factor |
|---|---|---|---|
| Butanoic acid | 2 | 34.98 ± 0.48 | 0.89 |
| Hexanoic acid | 4 | 36.00 ± 1.93 | 0.92 |
| Octanoic acid | 6 | 101.80 ± 25.9 | 2.60 |
| Decanoic acid | 8 | 159.12 ± 75.5 | 4.07 |
| Dodecanoic acid | 10 | 114.67 ± 13.7 | 2.93 |
| Tetradecanoic acid | 12 | 42.64 ± 2.02 | 1.09 |
| Octadecanoic acid | 16 | 41.44 ± 3.85 | 1.06 |

TABLE XIII

SKIN PERMEATION ENHANCEMENT OF NITROGLYCERIN BY SATURATED FATTY ACID PROPYL ESTERS

$$\overset{O}{\overset{//}{CH_3(CH_2)_nC-OCH_2CH_2CH_3}}$$

| Enhancers (1.6 mg/cm²) | n | Normalized Permeation Rate* (mcg/cm²/hr±S.D.) | Enhancing Factor |
|---|---|---|---|
| Hexanoic acid propyl ester | 4 | 43.67 ± 3.90 | 1.43 |
| Octanoic acid propyl ester | 6 | 49.69 ± 3.12 | 1.62 |
| Decanoic acid propyl ester | 8 | 69.02 ± 12.54 | 2.26 |
| Dodecanoic acid propyl ester | 10 | 58.59 ± 2.95 | 1.92 |
| Tetradecanoic acid propyl ester | 12 | 57.58 ± 6.54 | 1.88 |
| Hexadecanoic acid propyl ester | 14 | 48.11 ± 5.97 | 1.57 |
| Octadecanoic acid propyl ester | 16 | 51.02 ± 5.72 | 1.67 |

*Normalized Permeation Rate Without Enhancer = 30.58 ± 1.71 for Nitroglycerin

EXAMPLE 2

Following generally the procedure of Example 1, 15 percent W/W of nitroglycerin is incorporated into a polydimethylsiloxane sold under the designation Silastic Medical Grade 382 Elastomer to provide nitroglycerin containing matrix layer units. Also, following generally the procedure of Example 1, 15 percent W/W of nitroglycerin is incorporated into a polymethylvinylsiloxane sold under the designation Silastic Medical Grade MDX4-4210 to form nitroglycerin containing matrix layer units using a two-stage platinum catalyst for crosslinking. Skin permeation enhancers are incorporated into the adhesive compositions used to form the adhesive layer in the manner described in Example 1. The concentration of skin permeation enhancer used are shown in the following TABLES. As described in Example 1, the adhesive layer is formed by making

multiple coatings. At times, the adhesive coating solution (10-20%) is further diluted with regard to the skin permeation enhancer content by adding further adhesive polymer, which composition is applied to the release liner. The release liner surface with the adhesive coating then is applied to the adhesive layer which has been applied to the matrix surface to result in a unitary adhesive layer. When the release liner is removed when the final dosage unit is being prepared for application to the subject being treated, the thin coating of the adhesive having low content of skin permeation enhancer (usually no more than 1-2 percent enhancer) adheres satisfactorily to the skin of the subject being treated. The thickness of this layer is typically about 100 microns.

The skin permeation enhancer concentrations of the adhesive compositions making up the adhesive layers are shown in the following TABLES:

TABLE XIV

| SYNERGISTIC EFFECT IN SKIN PERMEABILITY ENHANCEMENT OF NITROGLYCERIN[1] | | | | |
|---|---|---|---|---|
| Enhancers composition (mg/cm$^2$) | | | Enhancement Factor | |
| (+) menthol | capric acid | 3rd enhancer | MDX4-4210 | DC-382 |
| 1.6 | -- | -- | 0.93 | -- |
| 0.8 | 0.8 | -- | 3.40 | -- |
| -- | 0.8 | -- | 2.26 | 2.80 |
| 0.8 | 0.8 | Decanol 0.8 | | 6.74 |
| 0.8 | 0.8 | Decanol 1.6 | 7.49 | 7.76 |
| 0.8 | 0.8 | Oleyl alcohol 0.8 | 6.13 | 5.29 |
| 0.8 | 0.8 | Oleyl alcohol 1.6 | 6.34 | 4.61 |
| 0.8 | 0.8 | Salicylic acid 0.8 | 4.01 | 3.47 |
| 0.8 | 0.8 | Methyl Salicylate 0.8 | 2.50 | 3.36 |

[1]15% W/W in either MDX4-4210 or DC-382 type silicone matrix.

TABLE XV

| SYNERGISTIC EFFECT IN SKIN PERMEABILITY ENHANCEMENT OF NITROGLYCERIN[1] | | | | | | | |
|---|---|---|---|---|---|---|---|
| Enhancers combination[3] (mg/cm$^2$) | | | | | | Enhancement Factor | |
| A | B | C | D | E | F | MDX4-4210[2] | DC-382 |
| 1.0 | 1.0 | -- | -- | -- | -- | 1.72 | -- |
| 1.0 | -- | 1.0 | -- | -- | -- | 3.32 | 2.96 |
| 1.0 | -- | -- | 1.0 | -- | -- | 3.10 | 3.45 |
| 1.0 | 1.0 | -- | -- | 1.6 | -- | 2.40 | 3.37 |
| 1.0 | -- | 1.0 | -- | 1.6 | -- | 16.68 | 7.24 |
| 1.0 | -- | -- | 1.0 | 1.6 | -- | 12.39 | -- |
| 1.0 | -- | -- | -- | 1.6 | -- | -- | 2.09 |
| -- | 1.0 | -- | -- | 1.6 | 1.0 | 4.04 | -- |
| -- | -- | 0.8 | -- | 1.6 | 0.8 | -- | 5.80 |

[1]Drug loading = 15% W/W
[2]Contains 6% dextran
[3]A = Decylmethyl sulfoxide
B = Octanol
C = Decanol
D = Oleyl alcohol
E = Squalane
F = Capric acid

TABLE XVI

| EFFECT OF ENHANCER LOCATION ON SKIN PERMEABILITY AND ENHANCEMENT OF NITROGLYCERIN BY PROPYL OLEATE | | | |
|---|---|---|---|
| Enhancer Concentration | | Permeation Rate (mcg/$cm^2$/hr) | Enhancement Factor |
| Matrix | Adhesive | | |
| 0% | 0% | 40.33 ± 6.04 | 1.00 |
| 2.5% | -- | 46.09 ± 5.07 | 1.14 |
| -- | 2.5% | 59.68 ± 17.34 | 1.48 |
| 2.5% | 2.5% | 71.00 ± 14` 48 | 1.76 |
| 5.0% | -- | 65.86 ± 22.61 | 1.63 |
| -- | 5.0% | 87.72 ± 16.31 | 2.18 |
| 5.0% | 5.0% | 109.58 ± 10.03 | 2.72 |
| -- | 10.0% | 125.86 ± 12.21 | 3.12 |

TABLE XVII

SYNERGISTIC EFFECT IN SKIN PERMEABILITY ENHANCEMENT OF NITROGLYCERIN[a)]

| Enhancers Composition: menthol[c)] ($mg/cm^2$) | Enhancers Composition: capric acid ($mg/cm^2$) | Enhancement Factor[b)] |
|---|---|---|
| (-) menthol | | |
| 1.6 | -- | 0.90 |
| 0.8 | 0.8 | 2.29 |
| (+) menthol | | |
| 1.6 | -- | 0.93 |
| 0.8 | 0.8 | 3.40 |
| (±) menthol | | |
| 1.6 | -- | 1.01 |
| 0.8 | 0.8 | 3.01 |
| -- | 0.8 | 2.26 |

[a)] 10% W/W in MDX4-4210-type silicone matrix.

[b)] Skin permeation rate for control device = 30.33 ± 0.2 $mcg/cm^2/hr$.

[c)] Menthol has been reportedly used to relieve local irritation or for counterirritant purposes.

TABLE XVIII

| SILICONE ELASTOMER COMPOSITION ON SKIN PERMEATION RATE OF NITROCLYCERIN | | |
|---|---|---|
| Drug Loading (% W/W) | Skin Permeation Rate (mcg/cm²/hr) | |
| | DC-382[a)] | MDX-4-4210[b)] |
| 10 | 37.79 ± 8.12 | 33.28 ± 4.77 |
| 15 | 33.05 ± 0.99 | 37.60 ± 11.87 |
| 20 | 30.14 ± 1.19 | 33.40 ± 4.44 |

[a)] Tin-catalyzed 2-part system
[b)] Platinum-catalyzed 2-part system

EXAMPLE 3

Dosage units are made using polymethylvinylsiloxane sold by Dow Corning under the designation MDX 4-4210 by following the general procedure described in Example 1. The polymer matrix layers have a nitroglycerin content of 12 percent and 6 percent of dextran based on the weight of the silicone polymer. The adhesive layer (thickness of about 300 microns) applied to the matrix layer has 3 percent squalane, 2.2 percent decylmethyl sulfoxide and 2.2 percent oleyl alcohol, based on the adhesive polymer weight. It is made by first applying to a release liner and then applying the coating to the matrix surface. The outer adhesive layer is applied to the release liner of the final dosage unit has 2 percent squalane, 1 percent decylmethyl sulfoxide and 1 percent oleyl alcohol; it has a thickness of about 100 microns. A laminating machine is employed to apply the respective adhesive layers to make the final matrix dosage unit. The dosage units show retention of nitroglycerin skin permeation rate after storage for one week at 25°, 37°, and 45°, respectively.

EXAMPLE 4

Examples 1-3 are essentially repeated using effective dosage amounts of isosorbate dinitrate, pentaerythrityl tritranitrate, erythrityl tetranitrate, and amyl nitrite respectively, instead of nitroglycerin.

## Claims

**1.** A transdermal anti-anginal pharmaceutical polymer matrix dosage unit comprising:

a) a backing layer which is substantially impervious to the pharmaceutical to be delivered transdermally;

b) a polymeric matrix disc layer which is adhered to said backing layer and which has microdispersed therein an amount of an anti-anginally effective pharmaceutical selected from pharmaceutically acceptable nitrate and nitrite esters of mono- and poly-hydric alcohols and combinations thereof but not including nitroglycerin, which will provide a dosage amount of said pharmaceutical; and

c) an adhesive layer which is adhered to said polymer matrix disc layer, and which has distributed therein an effective amount of one or more skin permeation enhancers which provide substantial skin absorption enhancement for said pharmaceutical;

said dosage unit having an enhancing factor of at least 1.2.

**2.** A dosage unit as claimed in claim 1, wherein said polymer matrix disc layer comprises a cross-linked polysiloxane polymer of the following formula:

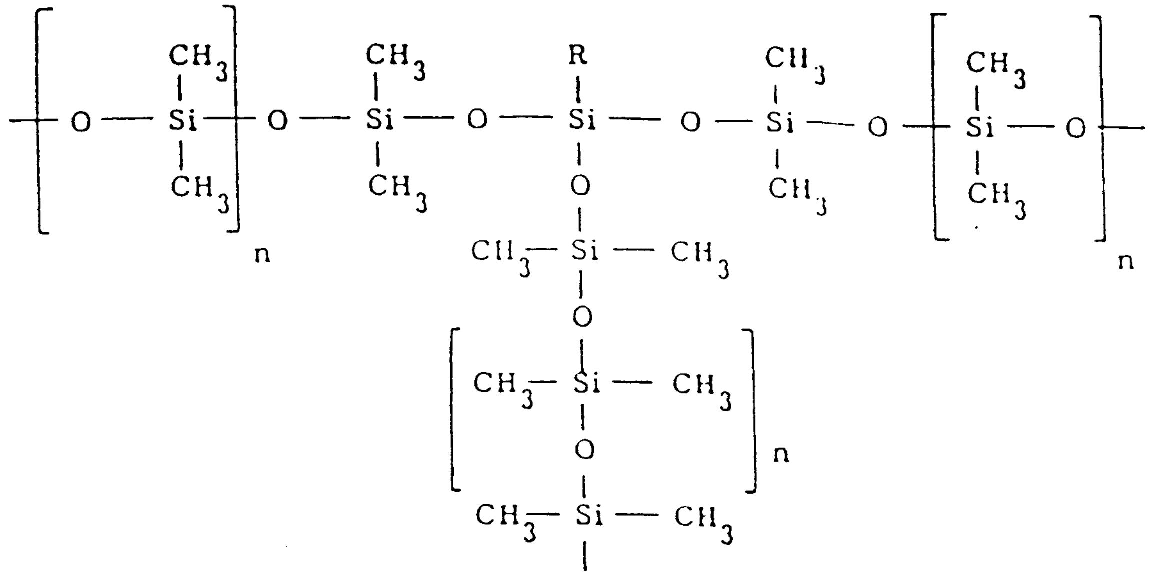

wherein R is alkyl or alkoxy having 1 - 7 carbon atoms, vinyl, phenyl or a combination thereof, and wherein n is 100 to 5,000.

**3.** A dosage unit as claimed in claim 1 or claim 2, wherein said polymer matrix disc layer comprises microdispersion compartments and has a cross sectional dimension of from 10 to 200 microns.

**4.** A dosage unit as claimed in any of the preceding claims wherein said enhancing factor is at least about 2.

**5.** A transdermal anti-anginal pharmaceutical polymer matrix dosage unit comprising:

a) a backing layer which is substantially impervious to the pharmaceutical to be delivered transdermally;

b) a polymeric matrix disc layer which has a thickness of from 0.1 to 5mm and which has microdispersed therein an anti-anginally effective pharmaceutical selected from pharmaceutically acceptable nitrate and nitrite esters of mono- and poly-hydric alcohols and combinations thereof but not including nitroglycerin, which will provide a dosage amount of said pharmaceutical, and is composed of a cross-linked polysiloxane polymer with said pharmaceutical in microdispersion compartments, and has a cross sectional dimension of from 10 to 200 microns;

c) a polysiloxane adhesive layer which is adhered to said polymer matrix disc layer, and which has distributed therein from 1 to 30 percent of one or more skin absorption enhancers based on the weight of the polysiloxane adhesive, which are effective in the enhancement of the transdermal absorption of said pharmaceutical in the polymer matrix disc layer;

said dosage unit having an enhancing factor of at least 1.2.

**6.** A dosage unit as claimed in claim 5 wherein the skin permeation enhancer is selected from decyl methyl sulfoxide, 1-dodecylaza-cycloheptan-2-one, a compound of the formula:

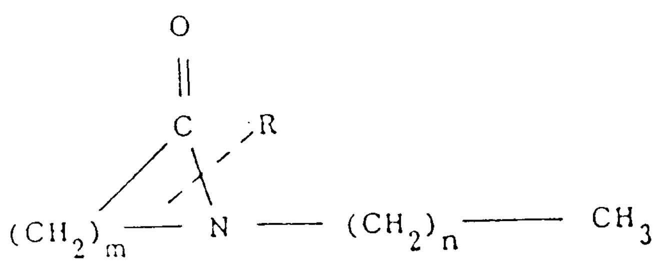

wherein R is H or a lower alkyl group, m is 5 - 7 and n is 0-17; saturated and unsaturated fatty acids and their alkyl esters, alcohols, monoglycerides, diethanolamides, triethanolamine complex, N,-N-dimethylamide derivatives, and acetate esters of said alcohol derivatives; propyl or isopropyl myristate; myristyl alcohol; propyl or isopropyl oleate; oleic acid; oleyl alcohol; oleyl acetate, monoolein; myristyl N, N-dimethyl amide; stearic acid; stearyl alcohol; propyl stearate; mono-stearin, mono-myristein; salicylic acid and derivatives; glycylglycine; N-N-diethyl-M-toluamide; squalane; capric acid; crotamiton; and combinations thereof.

**7.** A dosage unit as claimed in claim 5 wherein the skin permeation enhancer is selected from compounds of the following formula:

$CH_3(CH_2)_n$-COOH

wherein n is an integer from 6 to 10.

**8.** A dosage unit as claimed in any one of claims 5 to 7, wherein at least one skin permeation enhancer used is decanoic acid.

**9.** A dosage unit as claimed in claim 5, where the skin permeation enhancer is a combination comprising decanoic acid or decanol and 1-dodecylazacyclo-heptan-2-one.

**10.** A dosage unit as claimed in any one of claims 5 to 9, wherein both said matrix layer and said adhesive layer comprise effective amounts of one or more skin permeation enhancers.

**11.** A dosage unit as claimed in claim 5, wherein the skin permeation enhancer is a combination of 1-dodecylazacycloheptan-2-one, decanoic acid and decanol or oleyl alcohol.

**12.** A dosage unit as claimed in claim 5, wherein the skin permeation enhancer is a combination of squalane, decylmethyl sulfoxide and oleyl alcohol, or decanol.

**Revendications**

**1.** Dose unitaire à matrice polymère pour l'administration transdermique d'un produit pharmaceutique antiangineux comprenant :

a) une couche de support qui est sensiblement imperméable au produit pharmaceutique à délivrer par voie transdermique ;

b) une couche de matrice polymère en forme de disque, qui adhère à ladite couche de support et dans laquelle est microdispersée une quantité d'un produit pharmaceutique à action antiangineuse choisi parmi les nitrates et nitrites pharmaceutiquement acceptables de mono- et polyalcools et leurs associations, mais à l'exclusion de la nitroglycérine, qui peut fournir une dose dudit produit pharmaceutique ; et

c) une couche d'adhésif qui adhère à ladite couche de matrice polymère en forme de disque, et dans laquelle est répartie une quantité efficace d'un ou plusieurs activateurs de perméation cutanée, qui assurent une activation substantielle de l'absorption cutanée dudit produit pharmaceutique ;

ladite dosage unitaire ayant un facteur d'activation d'au moins 1,2.

**2.** Dose unitaire selon la revendication 1, dans laquelle ladite couche de matrice polymère en forme de disque comprend un polysiloxane réticulé répondant à la formule suivante :

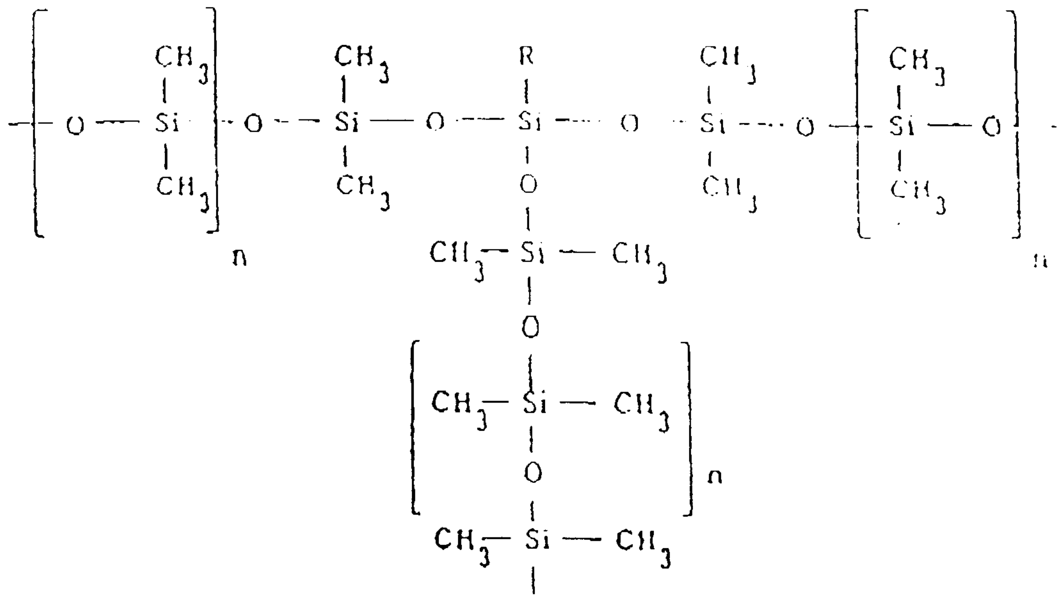

où R est un groupe alkyle ou alcoxy ayant 1 à 7 atomes de carbone, vinyle, phényle, ou une association de ceux-ci, et où n est de 100 à 5000.

3. Dose unitaire selon la revendication 1 ou la revendication 2, dans laquelle la couche de matrice polymère en forme de disque comprend des compartiments de microdispersion et a une dimension de 10 à 200 micromètres en section transversale.

4. Dose unitaire selon l'une quelconque des revendications précédentes, dans laquelle ledit facteur d'activation est d'du moins 2 environ.

5. Dose unitaire à matrice polymère pour l'administration transdermique d'un produit pharmaceutique antiangineux comprenant :
   a) une couche de support qui est sensiblement imperméable au produit pharmaceutique à délivrer par voie transdermique ;
   b) une couche de matrice polymère en forme de disque qui a une épaisseur de 0,1 à 5 mm et dans laquelle est microdispersé un produit pharmaceutique à action antiangineuse choisi parmi les nitrates et nitrites pharmaceutiquement acceptables de mono- et polyalcools et leurs associations, mais à l'exclusion de la nitroglycérine, qui peut fournir une dose dudit produit pharmaceutique, et qui est constituée d'un polysiloxane réticulé avec ledit produit pharmaceutique dans des compartiments de micro-dispersion et a une dimension de 10 à 200 micromètres en section transversale ;
   c) une couche d'adhésif de polysiloxane qui adhère à ladite couche de matrice polymère en forme de disque et dans laquelle sont répartis 1 à 30 pour cent d'un ou plusieurs activateurs d'absorption cutanée, par rapport au poids de l'adhésif de polysiloxane, qui sont efficaces pour activer l'absorption transdermique dudit produit pharmaceutique contenu dans la couche de matrice polymère en forme de disque,

   ladite dosage unitaire présentant un facteur d'activation d'au moins 1,2.

6. Dose unitaire selon la revendication 5, dans laquelle l'activateur de perméation cutanée est choisi parmi le décylméthylsulfoxyde, la 1-dodécylazacycloheptane-2-one, un composé de formule :

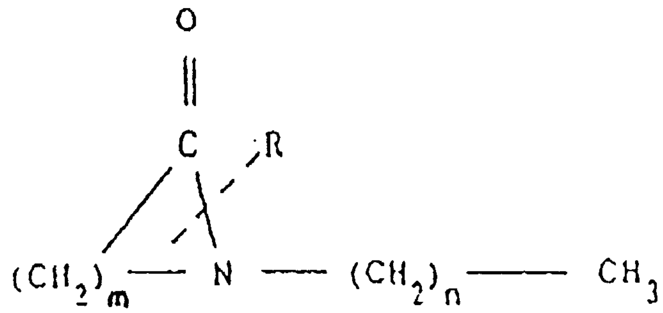

où R est H ou un groupe alkyle inférieur, m est de 5 à 7, et n est de 0 à 17 ; des acides gras saturés et insaturés et leurs dérivés de types esters d'alkyle, alcools, monoglycérides, diéthanolamides, complexe de triéthanolamine, dérivés de N,N-diméthylamide et esters acétiques desdits alcools ; le myristate de propyle ou d'isopropyle ; l'alcool myristylique ; l'oléate de propyle ou d'isopropyle ; l'acide oléique ; l'alcool oléylique ; l'acétate d'oléyle, la monooléine ; le myristyl-N,N-diméthylamide ; l'acide stéarique ; l'alcool stéarylique ; le stéarate de propyle ; la monostéarine, la mono-myristine ; l'acide salicylique et ses dérivés ; la glycylglycine ; le N,N-diéthyl-*m*-toluamide ; le squalane ; l'acide caprique ; le crotamiton ; et leurs associations.

7. Dose unitaire selon la revendication 5, dans laquelle l'activateur de perméation cutanée est choisi parmi les composés de formule suivante :

$CH_3(CH_2)_n$-COOH

où n est un nombre entier de 6 à 10.

8. Dose unitaire selon l'une quelconque des revendications 5 à 7, dans laquelle au moins un activateur de perméation cutanée utilisé est l'acide décanoïque.

9. Dose unitaire selon la revendication 5, dans laquelle l'activateur de perméation cutanée est une association comprenant de l'acide décanoïque ou du décanol et de la 1-dodécylazacycloheptane-2-one.

10. Dose unitaire selon l'une quelconque des revendications 5 à 9, dans laquelle ladite couche de matrice et ladite couche d'adhésif comprennent toutes deux des quantités efficaces d'un ou plusieurs activateurs de perméation cutanée.

11. Dose unitaire selon la revendication 5, dans laquelle l'activateur de perméation cutanée est une association de 1-dodécylazacycloheptane-2-one, d'acide décanoïque et de décanol ou d'alcool oléylique.

12. Dose unitaire selon la revendication 5, dans laquelle l'activateur de perméation cutanée est une association de squalane, de décylméthylsulfoxyde et d'alcool oléylique ou de décanol.

**Patentansprüche**

1. Transdermale, anti-anginale, pharmazeutische Polymermatrix-Dosiseinheit, umfassend
   (a) eine Verstärkungsschicht, die für das transdermal abzugebende Arzneimittel im wesentlichen undurchlässig ist;
   (b) eine Polymermatrix-Lamellenschicht, die an der Verstärkungsschicht haftet und die,hierin mikrodispergiert,eine Menge eines anti-anginal wirksamen Arzneimittels, ausgewählt unter pharmazeutisch verträglichen Nitrat- und Nitritestern der ein- und mehrwertigen Alkohole und Kombinationen hiervon, jedoch nicht eingeschlossen Nitroglycerin, enthält, die eine Dosismenge des Arzneimittels ergibt; und
   (c) eine Haftschicht, die an der Polymermatrix-Lamellenschicht haftet und die, hierin verteilt, eine wirksame Menge eines oder mehrerer Hautpermeationsverstärker enthält, die eine wesentliche Hautabsorptionsverstärkung für das Arzneimittel ergibt;

wobei die Dosiseinheit einen Verstärkungsfaktor von zumindest 1,2 aufweist.

**2.** Dosiseinheit gemäß Anspruch 1, worin die Polymermatrix-Lamellenschicht ein vernetztes Polysiloxanpolymeres der folgenden Formel

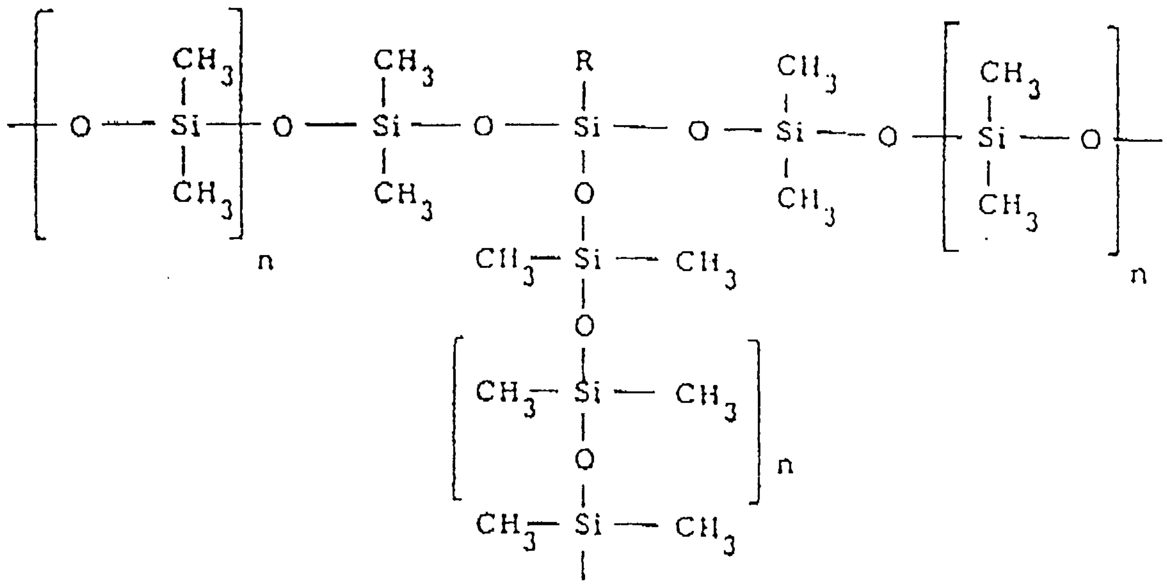

worin R für Alkyl oder Alkoxy mit 1 bis 7 Kohlenstoffatomen, Vinyl, Phenyl oder eine Kombination hiervon steht und worin n 100 bis 5000 beträgt, umfaßt.

**3.** Dosiseinheit gemäß Anspruch 1 oder 2, worin die Polymermatrix-Lamellenschicht Mikrodispersions-Zwischenräume umfaßt und eine Querschnittsabmessung von 10 bis 200 Mikron aufweist.

**4.** Dosiseinheit gemäß einem der vorhergehenden Ansprüche, worin der Verstärkungsfaktor zumindest etwa 2 beträgt.

**5.** Transdermale, anti-anginale, pharmazeutische Polymermatrix-Dosiseinheit, umfassend
(a) eine Verstärkungsschicht, die gegenüber dem transdermal freizugebenden Arzneimittel im wesentlichen undurchlässig ist;
(b) eine Polymermatrix-Lamellenschicht, die eine Dicke von 0,1 bis 5 mm besitzt und die, hierin mikrodispergiert, ein anti-anginal wirksames Arzneimittel, ausgewählt unter pharmazeutisch verträglichen Nitrat- und Nitritestern der ein- und mehrwertigen Alkohole und Kombinationen hiervon, jedoch nicht unter Einschluß von Nitroglycerin, enthält, die eine Dosismenge des Arzneimittels ergibt und aus einem vernetzten Polysiloxanpolymeren mit dem Arzneimittel in Mikrodispersions-Zwischenräumen besteht und eine Querschnittsabmessung von 10 bis 200 Mikron besitzt;
(c) eine Polysiloxan-Haftschicht, die an der Polymermatrix-Lamellenschicht haftet und die, hierin verteilt, 1 bis 30% eines oder mehrerer Hautabsorptionsverstärker, bezogen auf das Gewicht des Polysiloxan-Haftmittels, enthält, die im Hinblick auf die Verstärkung der transdermalen Absorption des Arzneimittels in der Polymermatrix-Lamellenschicht wirksam sind;
wobei die Dosiseinheit einen Verstärkungsfaktor von zumindest 1,2 besitzt.

**6.** Dosiseinheit gemäß Anspruch 5, worin der Hautpermeationsverstärker unter Decylmethylsulfoxid, 1-Dodecylaza-cycloheptan-2-on, einer Verbindung der Formel

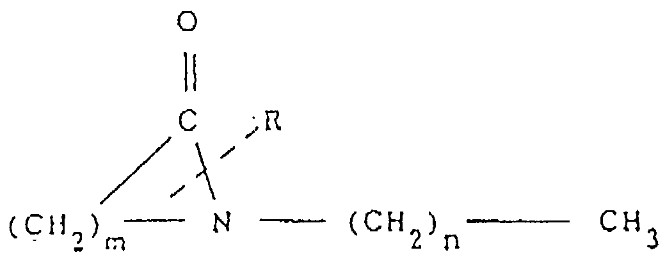

worin R für H oder eine Niedrigalkylgruppe steht, m 5 bis 7 ist und n 0 bis 17 ist, gesättigten oder ungesättigten Fettsäuren und ihren Alkylestern, Alkoholen, Monoglyceriden, Diethanolamiden, Triethanolamin-Komplex, N,N-Dimethylamidderivaten und Acetatestern dieser Alkoholderivate; Propyl- oder Isopropylmyristat; Myristylalkohol; Propyl- oder Isopropyloleat; Ölsäure; Oleylalkohol; Oleylacetat, Monoolein; Myristyl-N,N-dimethylamid; Stearinsäure; Stearylalkohol; Propylstearat, Monostearin, Monomyristein; Salicylsäure und Derivate; Glycylglycin; N,N-Diethyl-m-toluamid; Squalan; Caprinsäure; Crotamiton; und Kombinationen hiervon ausgewählt ist.

**7.** Dosiseinheit gemäß Anspruch 5, worin der Hautpermeationsverstärker unter Verbindungen der folgenden Formel

$CH_3(CH_2)_n$-COOH

worin n für eine ganze Zahl von 6 bis 10 steht, ausgewählt ist.

**8.** Dosiseinheit gemäß einem der Ansprüche 5 bis 7, worin zumindest ein Hautpermeationsverstärker Decansäure ist.

**9.** Dosiseinheit gemäß Anspruch 5, worin der Hautpermeationsverstärker eine Kombination, umfassend Decansäure oder Decanol und 1-Dodecylazacyclo-heptan-2-on, ist.

**10.** Dosiseinheit gemäß einem der Ansprüche 5 bis 9, worin sowohl die Matrixschicht als auch die Haftschicht wirksame Mengen eines oder mehrerer Hautpermeationsverstärker enthalten.

**11.** Dosiseinheit gemäß Anspruch 5, worin der Hautpermeationsverstärker eine Kombination von 1-Dodecylazacyclo-heptan-2-on, Decansäure und Decanol oder Oleylalkohol ist.

**12.** Dosiseinheit gemäß Anspruch 5, worin der Hautpermeationsverstärker eine Kombination von Squalan, Decylmethylsulfoxid und Oleylalkohol oder Decanol ist.